# EUROPEAN PATENT APPLICATION

(11) **EP 4 810 464 A1**
(43) Date of publication of application: **23.09.2026**
(21) Application number: 25902423.0
(22) Date of filing: 04.11.2025
(51) Int. Cl.: C07C 17/361, C07C 17/20, C07C 21/18, B01J 23/83, B01J 23/10, B01J 23/26, B01J 23/28

(54) **PRODUCTION OF 1234YF BY MEANS OF ONE-STEP FLUORINATION OF 240DB**

(30) Priority: 24.07.2025 CN 202511024633
(71) Applicant: Zhejiang Quhua Fluor-Chemistry Co., Ltd., Quzhou, Zhejiang 324004 (CN); Zhejiang Juhua Technology Center Co., Ltd., Quzhou, Zhejiang 324004 (CN)
(72) Inventor: LIU, Bin, Quzhou, Zhejiang 324004 (CN); GE, Wenfeng, Quzhou, Zhejiang 324004 (CN); ZHANG, Yan, Quzhou, Zhejiang 324004 (CN); ZHAO, Yang, Quzhou, Zhejiang 324004 (CN); OUYANG, Xuhui, Quzhou, Zhejiang 324004 (CN); TONG, Chaoli, Quzhou, Zhejiang 324004 (CN); ZHANG, Qidong, Quzhou, Zhejiang 324004 (CN); REN, Yawen, Quzhou, Zhejiang 324004 (CN); ZHU, Qinglong, Quzhou, Zhejiang 324004 (CN); QIU, Zhengyi, Quzhou, Zhejiang 324004 (CN); WU, Tianyong, Quzhou, Zhejiang 324004 (CN)
(74) Representative: karo IP
(86) International application number: PCT/CN2025/132355
(87) International publication number: WO 2026/138179

(57) **Abstract**

Disclosed in the present invention is a method for one-step fluorination of 240db to generate 1234yf, which includes: allowing 240db and hydrogen fluoride to undergo a one-step reaction under the action of a catalyst subjected to fluorination treatment to generate 1234yf, where the catalyst includes a Ce-Zr composite oxide support and one or two or more of Ni, La, Cr, Co, Fe, Sb, Mo, and Cu supported on the support. The method of the present invention has the characteristics of a stable and controllable process and a long catalyst service life while ensuring high selectivity and high yield, thereby effectively reducing material consumption and equipment maintenance costs in industrial production, and solving the problems of economy and stability of the prior art in large-scale production.

## Description

### TECHNICAL FIELD

The present invention relates to the technical field of synthesis of 2,3,3,3-tetrafluoropropene (1234yf), and particularly relates to a method for one-step fluorination of 1,1,1,2,3-pentachloropropane (240db) to generate 1234yf.

### BACKGROUND

With continuous evolution of refrigeration technologies, the search for efficient, environmentally friendly, and economical refrigerants has become a focal point in the industry. With the increasing global awareness of environmental protection, reducing greenhouse gas emissions and ozone layer depletion has become an international consensus. Against this background, 2,3,3,3-tetrafluoropropene (referred to as HFO-1234yf), with its extremely low global warming potential (GWP < 1), zero ozone depletion potential (ODP = 0), and short atmospheric lifetime (only 11 days), has been recognized as an ideal substitute for traditional refrigerants such as HFC-134a, marking the fourth generation of innovation in the refrigerant industry.

However, although exhibiting significant environmental advantages, HFO-1234yf faces numerous challenges in current industrial production routes. Existing technologies mainly rely on synthesis routes with multiple steps and high energy consumption, such as complex conversion processes using hexafluoropropene, trifluoropropene, tetrachloropropene, and the like as starting materials.

For example, a continuous preparation method for 2,3,3,3-tetrafluoropropene is reported in patent CN109796300B, in which carbon tetrachloride and ethylene are used as raw materials. CCl₄ and the ethylene undergo telomerization to generate CCl₃CH₂CH₂Cl, then CCl₃CH₂CH₂Cl undergoes dehydrochlorination to form CCl₂=CHCH₂Cl, CCl₂=CHCH₂Cl is then chlorinated to obtain CCl₃CHClCH₂Cl, CCl₃CHClCH₂Cl undergoes dehydrochlorination to obtain CCl₂=CClCH₂Cl, CCl₂=CClCH₂Cl then reacts with hydrogen fluoride to generate 2-chloro-3,3,3-trifluoropropene (CF₃CCl=CH₂, 1233xf), and CF₃CCl=CH₂ undergoes eilfluorine-chlorine exchange to obtain the final product, HFO-1234yf.

For another example, formation of HFO-1234yf is reported in patent application CN117377646A, in which CCl₃CH₂CH₂Cl undergoes a fluorination reaction to form CF₃CH=CH₂, CF₃CH=CH₂ undergoes a chlorination reaction to form CF₃CHClCH₂Cl, CF₃CHClCH₂Cl undergoes a dehydrochlorination reaction to form CF₃CCl=CH₂, CF₃CCl=CH₂ contacts with hydrogen fluoride in the presence of a catalyst and undergoes a hydrofluorination reaction to form CF₃CFClCH₃, and CF₃CFClCH₃ undergoes a dehydrochlorination reaction to finally form HFO-1234yf. This route requires a total of 6 reaction steps, which not only results in a lengthy process, but also demands stringent reaction conditions, especially operations at high temperatures, thereby not only increasing equipment investment costs, but also significantly increasing the energy consumption level.

The specification of a patent with publication No. CN107074697A discloses a method for producing 2,3,3,3-tetrafluoropropene, which includes: allowing a composition containing at least 99 wt% of 1,1,1,2,3-pentachloropropane to react with hydrofluoric acid in a gas phase, which may be a catalytic fluorination step. A supported or unsupported catalyst containing a metal such as Cr, Ni, Fe, Zn, Ti, V, Zr, Mo, Ge, Sn, Pb, Mg, or Sb, chromium oxyfluoride, aluminum fluoride, or aluminum oxyfluoride may be used as a catalyst.

### SUMMARY

The present invention provides a method for one-step fluorination of 240db to generate 1234yf, which simplifies a process flow, improves reaction effects, and can solve the problems of complicated processes, multiple intermediates, low yield of final product 1234yf, and easy catalyst deactivation in the prior art.

Specific technical solutions are as follows.

A method for one-step fluorination of 240db to generate 1234yf includes: allowing 240db and hydrogen fluoride to undergo a one-step reaction under the action of a catalyst subjected to fluorination treatment to generate 1234yf, where
the catalyst includes a Ce-Zr composite oxide support and one or two or more of Ni, La, Cr, Co, Fe, Sb, Mo, and Cu supported on the support. Further, the metal supported on the support preferably includes one or two or more of Ni, Cr, Co, and Cu, which has excellent activation capability for a fluorine source (such as HF, etc.), and can effectively promote the reaction between 240db and fluorine to generate 1234yf in one step.

The fluorination treatment can convert active components of the catalyst into fluorides, while forming a fluorine-oxygen layer on the surface of the Ce-Zr composite oxide support.

In the method for one-step fluorination of 240db to generate 1234yf, the fluorination treatment preferably includes: subjecting the catalyst to a fluorination reaction in a gas containing HF at 280-360°C (e.g., 300°C, 340°C, 350°C, etc., preferably 300-350°C).

Further, the gas containing HF may include HF and an inert gas.

In the present invention, the inert gas refers to a gas that does not participate in the reaction, such as nitrogen, etc.

In some preferred examples, a volume fraction of HF in the gas containing HF is 5%-10%.

In some preferred examples, a time of the fluorination reaction is 2-3 hours.

A method for preparing the catalyst preferably includes: by means of an impregnation method, immersing the Ce-Zr composite oxide support in a precursor solution containing one or two or more of Ni, La, Cr, Co, Fe, Sb, Mo, and Cu, and after completion of the immersing, taking out a solid, followed by drying and calcination to obtain the catalyst. A temperature of the calcination is preferably 340-400°C, such as 350°C, 380°C, etc., further preferably 350-380°C. A time of the calcination is preferably 3-4 hours. The precursor solution may be a salt solution, etc., and further may be a nitrate solution, a chloride solution, etc.

A method for preparing the Ce-Zr composite oxide support preferably includes: by means of a co-precipitation-hydrothermal method, adjusting a pH of a solution containing Ce(III) and Zr(IV) to 9-10, then carrying out a hydrothermal reaction at 160-200°C (e.g., 180°C, 190°C, etc., preferably 180-190°C), and after completion of the hydrothermal reaction, taking out a solid, followed by calcination to obtain the Ce-Zr composite oxide support. A time of the hydrothermal reaction is preferably 12-18 hours. A temperature of the calcination is preferably 480-520°C, further preferably 500°C. A time of the calcination is preferably 4-5 hours.

In the method for preparing the Ce-Zr composite oxide support, Ce(III) is preferably derived from cerium nitrate.

In the method for preparing the Ce-Zr composite oxide support, Zr(IV) is preferably derived from zirconium nitrate.

In the method for preparing the Ce-Zr composite oxide support, ammonia is preferably used to adjust the pH.

In the catalyst, based on a mass of the Ce-Zr composite oxide support as 100%, a mass proportion of one or two or more of Ni, La, Cr, Co, Fe, Sb, Mo, and Cu is preferably 5%-15%, such as 10%, etc.

In the Ce-Zr composite oxide support, a molar ratio of Ce to Zr is preferably 1:(1-5), such as 1:3, etc.

In the method for one-step fluorination of 240db to generate 1234yf, a molar ratio of 240db to the hydrogen fluoride is preferably 1:(5-30), such as 1:10, 1:15, 1:20, 1:25, 1:30, etc.

In the method for one-step fluorination of 240db to generate 1234yf, a reaction temperature of the one-step reaction to generate 1234yf is preferably 250-390°C, such as 300°C, etc.

In the method for one-step fluorination of 240db to generate 1234yf, a reaction pressure of the one-step reaction to generate 1234yf is preferably 0.1-0.6 MPa, such as 0.4 MPa, etc.

In the method for one-step fluorination of 240db to generate 1234yf, a volumetric hourly space velocity of the catalyst for the one-step reaction to generate 1234yf is preferably 500-1,500 h⁻¹, such as 1,000 h⁻¹, etc.

As a general inventive concept, the present invention further provides a catalyst, a preparation method therefor, and use thereof. The catalyst and the preparation method therefor may refer to the descriptions in the above method for one-step fluorination of 240db to generate 1234yf. The catalyst can be used for catalyzing a one-step reaction of 240db and hydrogen fluoride to generate 1234yf. Preferably, the catalyst is subjected to fluorination treatment before being used for catalyzing the one-step reaction of 240db and hydrogen fluoride to generate 1234yf, and the fluorination treatment may refer to the descriptions in the above method for one-step fluorination of 240db to generate 1234yf.

The catalyst subjected to fluorination treatment according to the present invention has the following characteristics.

### 1. Sintering resistance:

Solid solution stabilization: By adopting a co-precipitation-hydrothermal-calcination method, Ce⁴⁺ is formed, and Ce⁴⁺ is allowed to enter a ZrO₂ lattice, thereby increasing lattice distortion energy and inhibiting grain growth at high temperatures.

Strong metal-support interaction: Active components are converted into fluorides, which form chemical bonding with a Ce-Zr composite oxide support interface that forms a fluorine-oxygen layer on a surface, thereby limiting migration and aggregation of the active components. Due to the strong metal-support interaction, on one hand, the yield and selectivity of 1234yf are greatly increased, and on the other hand, the catalyst has an ultra-long lifetime.

### 2. Carbon deposition resistance:

Surface acidity regulation: A carbon deposition precursor is mainly polymerized at strong acid sites. A cerium oxide in the catalyst of the present invention can reduce the density of strong acid sites on the support surface, thereby reducing the adsorption of olefinic substances.

Oxidative removal effect: Due to the characteristic of an oxygen storage capacity of the cerium oxide, deposited carbon species can be oxidized into CO, CO₂, etc., thereby inhibiting the generation of carbon deposition.

Compared with the prior art, the present invention has the following beneficial effects.
(1) By constructing the reaction system for one-step fluorination of 240db to prepare 1234yf, deep integration of traditional multi-step reaction processes is achieved, thereby effectively shortening a reaction pathway, reducing the complexity of separation and purification of intermediate products, and laying a structural foundation for improving reaction efficiency.
(2) According to the use of the specific catalyst, the generation selectivity of 1234yf and the yield of the target product are significantly increased. Meanwhile, by enhancing an anti-deactivation capability of the catalyst, its stable operation period is greatly prolonged, thereby solving the technical bottlenecks of rapid activity decay and difficulty in product distribution control in conventional catalytic processes.
(3) The method of the present invention has the characteristics of a stable and controllable process and a long catalyst service life while ensuring high selectivity and high yield, thereby effectively reducing material consumption and equipment maintenance costs in industrial production, and solving the problems of economy and stability of the prior art in large-scale production.

### DETAILED DESCRIPTION OF THE EMBODIMENTS

The present invention is further illustrated below in combination with specific examples. It should be understood that these examples are merely used for illustrating the present invention and are not intended to limit the scope of the present invention.

Operation methods without specific conditions in the following examples are generally used according to conventional conditions or according to conditions recommended by manufacturers.

### Example 1:

By means of a co-precipitation-hydrothermal method, a solution containing cerium nitrate and zirconium nitrate was adjusted to a pH of 9-10 with ammonia, then a hydrothermal reaction was carried out at 180°C for 12 hours, and after completion of the hydrothermal reaction, a solid was taken out and calcined at 500°C for 4 hours to obtain a Ce-Zr composite oxide support. In the Ce-Zr composite oxide support, a molar ratio of Ce to Zr was 1:1.

By means of an impregnation method, the Ce-Zr composite oxide support obtained above was immersed in a nickel nitrate solution, and after completion of the immersing, a solid was taken out, dried, and calcined at 350°C for 3 hours to obtain a Ni catalyst. In the Ni catalyst, based on a mass of the Ce-Zr composite oxide support as 100%, a mass proportion of Ni was 5%.

The Ni catalyst obtained above was allowed to undergo a fluorination reaction in an HF/N₂ mixed gas containing 5 vol% of HF at 340°C for 2 hours to obtain a Ni catalyst subjected to fluorination treatment.

240db and hydrogen fluoride were allowed to undergo a one-step reaction under the action of the above Ni catalyst subjected to fluorination treatment to generate 1234yf. Reaction parameter conditions such as reaction pressure, reaction temperature, and raw material molar ratio, as well as the composition of an outlet gas after 20 hours of the reaction are shown in Table 1.

### Example 2:

By means of a co-precipitation-hydrothermal method, a solution containing cerium nitrate and zirconium nitrate was adjusted to a pH of 9-10 with ammonia, then a hydrothermal reaction was carried out at 180°C for 12 hours, and after completion of the hydrothermal reaction, a solid was taken out and calcined at 500°C for 4 hours to obtain a Ce-Zr composite oxide support. In the Ce-Zr composite oxide support, a molar ratio of Ce to Zr was 1:3.

By means of an impregnation method, the Ce-Zr composite oxide support obtained above was immersed in a lanthanum nitrate solution, and after completion of the immersing, a solid was taken out, dried, and calcined at 350°C for 3 hours to obtain a La catalyst. In the La catalyst, based on a mass of the Ce-Zr composite oxide support as 100%, a mass proportion of La was 10%.

The La catalyst obtained above was allowed to undergo a fluorination reaction in an HF/N₂ mixed gas containing 5 vol% of HF at 340°C for 2 hours to obtain a La catalyst subjected to fluorination treatment.

240db and hydrogen fluoride were allowed to undergo a one-step reaction under the action of the above La catalyst subjected to fluorination treatment to generate 1234yf. Reaction parameter conditions such as reaction pressure, reaction temperature, and raw material molar ratio, as well as the composition of an outlet gas after 20 hours of the reaction are shown in Table 1.

### Example 3:

By means of a co-precipitation-hydrothermal method, a solution containing cerium nitrate and zirconium nitrate was adjusted to a pH of 9-10 with ammonia, then a hydrothermal reaction was carried out at 180°C for 12 hours, and after completion of the hydrothermal reaction, a solid was taken out and calcined at 500°C for 4 hours to obtain a Ce-Zr composite oxide support. In the Ce-Zr composite oxide support, a molar ratio of Ce to Zr was 1:5.

By means of an impregnation method, the Ce-Zr composite oxide support obtained above was immersed in a chromium chloride solution, and after completion of the immersing, a solid was taken out, dried, and calcined at 350°C for 3 hours to obtain a Cr catalyst. In the Cr catalyst, based on a mass of the Ce-Zr composite oxide support as 100%, a mass proportion of Cr was 15%.

The Cr catalyst obtained above was allowed to undergo a fluorination reaction in an HF/N₂ mixed gas containing 5 vol% of HF at 340°C for 2 hours to obtain a Cr catalyst subjected to fluorination treatment.

240db and hydrogen fluoride were allowed to undergo a one-step reaction under the action of the above Cr catalyst subjected to fluorination treatment to generate 1234yf. Reaction parameter conditions such as reaction pressure, reaction temperature, and raw material molar ratio, as well as the composition of an outlet gas after 20 hours of the reaction are shown in Table 1.

**Table 1**

| Example | Catalyst | Reaction temperature (°C) | Reaction pressure (MPa) | Volumetric hourly space velocity (h⁻¹) | Molar ratio of hydrogen fluoride to 240db | Molar composition of an outlet gas /% | | |
|---|---|---|---|---|---|---|---|---|
| | | | | | | 1234yf | 1233xf | Other substance |
| Example 1 | Ni catalyst | 250 | 0.1 | 500 | 5:1 | 16.6 | 68.2 | 15.2 |
| Example 2 | La catalyst | 300 | 0.4 | 1000 | 15:1 | 35.3 | 51.1 | 13.6 |
| Example 3 | Cr catalyst | 390 | 0.6 | 1500 | 30:1 | 44.7 | 47.2 | 8.1 |

### Example 4:

The only difference from Example 1 is that a cobalt nitrate solution was used to replace the nickel nitrate solution during immersing to obtain a Co catalyst. In the Co catalyst, based on a mass of a Ce-Zr composite oxide support as 100%, a mass proportion of Co was 5%. All other conditions are the same. Reaction parameter conditions such as reaction pressure, reaction temperature, and raw material molar ratio for one-step fluorination of 240db to generate 1234yf, as well as the composition of an outlet gas after 20 hours of the reaction are shown in Table 2.

### Example 5:

The only difference from Example 2 is that chromium nitrate and copper nitrate solutions (a molar ratio of chromium to copper was 1:1) were used to replace the lanthanum nitrate solution during immersing to obtain a Cr-Cu catalyst. In the Cr-Cu catalyst, based on a mass of a Ce-Zr composite oxide support as 100%, a total mass proportion of Cr and Cu was 10%, with an equal molar ratio of Cr to Cu. All other conditions are the same. Reaction parameter conditions such as reaction pressure, reaction temperature, and raw material molar ratio for one-step fluorination of 240db to generate 1234yf, as well as the composition of an outlet gas after 20 hours of the reaction are shown in Table 2.

### Example 6:

The only difference from Example 3 is that iron nitrate and molybdenum nitrate solutions (a molar ratio of iron to molybdenum was 1:1) were used to replace the chromium chloride solution during immersing to obtain an Fe-Mo catalyst. In the Fe-Mo catalyst, based on a mass of a Ce-Zr composite oxide support as 100%, a total mass proportion of Fe and Mo was 15%, with an equal molar ratio of Fe to Mo. All other conditions are the same. Reaction parameter conditions such as reaction pressure, reaction temperature, and raw material molar ratio for one-step fluorination of 240db to generate 1234yf, as well as the composition of an outlet gas after 20 hours of the reaction are shown in Table 2.

**Table 2**

| Example | Catalyst | Reaction temperature (°C) | Reaction pressure (MPa) | Volumetric hourly space velocity (h⁻¹) | Molar ratio of hydrogen fluoride to 240db | Molar composition of an outlet gas /% | | |
|---|---|---|---|---|---|---|---|---|
| | | | | | | 1234yf | 1233xf | Other substance |
| Example 4 | Co catalyst | 250 | 0.1 | 500 | 5:1 | 18.9 | 68.6 | 12.5 |
| Example 5 | Cr-Cu catalyst | 300 | 0.4 | 1000 | 15:1 | 43.1 | 46.3 | 10.6 |
| Example 6 | Fe-Mo catalyst | 390 | 0.6 | 1500 | 30:1 | 45.2 | 48.3 | 6.5 |

### Example 7:

Referring to Example 5, a long-term experiment of one-step fluorination of 240db to generate 1234yf was conducted, with a total catalytic reaction time of 500 hours. Results are shown in Table 3.

**Table 3**

| Example | Reaction time (h) | Molar composition of an outlet gas /% | | |
|---|---|---|---|---|
| | | 1234yf | 1233xf | Other substance |
| | 100 | 43.8 | 45.3 | 10.9 |
| | 200 | 42.5 | 46.1 | 11.4 |
| Example 7 | 300 | 44.1 | 45.8 | 10.1 |
| | 400 | 43.6 | 45.6 | 10.8 |
| | 500 | 43.8 | 46.6 | 9.6 |

It can be seen that the catalyst of the present invention exhibits a strong anti-deactivation capability, which maintains activity without decline after catalyzing the reaction continuously for 500 hours, thus having an excellent industrial application value.

It should also be understood that after reading the above descriptions of the contents of the present invention, those skilled in the art may make various alterations or modifications to the present invention, and such equivalent forms shall also fall within the scope defined by the appended claims of the present application.

## Claims

1. A method for one-step fluorination of 240db to generate 1234yf, comprising: allowing 240db and hydrogen fluoride to undergo a one-step reaction under the action of a catalyst subjected to fluorination treatment to generate 1234yf, wherein
the catalyst comprises a Ce-Zr composite oxide support and one or two or more of Ni, La, Cr, Co, Fe, Sb, Mo, and Cu supported on the support.

2. The method for one-step fluorination of 240db to generate 1234yf according to claim 1, wherein the fluorination treatment comprises: subjecting the catalyst to a fluorination reaction in a gas containing HF at 280-360°C.

3. The method for one-step fluorination of 240db to generate 1234yf according to claim 2, wherein the fluorination treatment comprises: subjecting the catalyst to the fluorination reaction in the gas containing HF at 300-350°C.

4. The method for one-step fluorination of 240db to generate 1234yf according to claim 2 or 3, wherein the gas containing HF comprises HF and an inert gas.

5. The method for one-step fluorination of 240db to generate 1234yf according to claim 2 or 3, wherein a volume fraction of HF in the gas containing HF is 5%-10%.

6. The method for one-step fluorination of 240db to generate 1234yf according to claim 2 or 3, wherein a time of the fluorination reaction is 2-3 hours.

7. The method for one-step fluorination of 240db to generate 1234yf according to claim 1, wherein a method for preparing the catalyst comprises: by means of an impregnation method, immersing the Ce-Zr composite oxide support in a precursor solution containing one or two or more of Ni, La, Cr, Co, Fe, Sb, Mo, and Cu, and after completion of the immersing, taking out a solid, followed by drying and calcination to obtain the catalyst.

8. The method for one-step fluorination of 240db to generate 1234yf according to claim 7, wherein a temperature of the calcination is 340-400°C.

9. The method for one-step fluorination of 240db to generate 1234yf according to claim 8, wherein the temperature of the calcination is 350-380°C.

10. The method for one-step fluorination of 240db to generate 1234yf according to any one of claims 7-9, wherein a time of the calcination is 3-4 hours.

11. The method for one-step fluorination of 240db to generate 1234yf according to claim 1, wherein a method for preparing the Ce-Zr composite oxide support comprises: by means of a co-precipitation-hydrothermal method, adjusting a pH of a solution containing Ce(III) and Zr(IV) to 9-10, then carrying out a hydrothermal reaction at 160-200°C, and after completion of the hydrothermal reaction, taking out a solid, followed by calcination to obtain the Ce-Zr composite oxide support.

12. The method for one-step fluorination of 240db to generate 1234yf according to claim 11, wherein the hydrothermal reaction is carried out at 180-190°C.

13. The method for one-step fluorination of 240db to generate 1234yf according to claim 11 or 12, wherein a time of the hydrothermal reaction is 12-18 hours.

14. The method for one-step fluorination of 240db to generate 1234yf according to claim 11, wherein a temperature of the calcination is 480-520°C, and a time of the calcination is 4-5 hours.

15. The method for one-step fluorination of 240db to generate 1234yf according to claim 1, wherein in the catalyst, based on a mass of the Ce-Zr composite oxide support as 100%, a mass proportion of one or two or more of Ni, La, Cr, Co, Fe, Sb, Mo, and Cu is 5%-15%.

16. The method for one-step fluorination of 240db to generate 1234yf according to claim 1 or 15, wherein in the Ce-Zr composite oxide support, a molar ratio of Ce to Zr is 1:(1-5).

17. The method for one-step fluorination of 240db to generate 1234yf according to claim 1, wherein a molar ratio of 240db to the hydrogen fluoride is 1:(5-30).

18. The method for one-step fluorination of 240db to generate 1234yf according to claim 1, wherein a reaction temperature of the one-step reaction to generate 1234yf is 250-390°C.

19. The method for one-step fluorination of 240db to generate 1234yf according to claim 1, wherein a reaction pressure of the one-step reaction to generate 1234yf is 0.1-0.6 MPa.

20. The method for one-step fluorination of 240db to generate 1234yf according to claim 1, wherein a volumetric hourly space velocity of the catalyst for the one-step reaction to generate 1234yf is 500-1,500 h⁻¹.
